(19) Europäisches Patentamt

European Patent Office

Office européen des brevets

(11) **EP 0 551 043 B1**

## (12) FASCICULE DE BREVET EUROPEEN

(45) Date de publication et mention
de la délivrance du brevet:
**10.07.1996 Bulletin 1996/28**

(51) Int. Cl.⁶: $A61M\ 1/14$

(21) Numéro de dépôt: **92403573.6**

(22) Date de dépôt: **29.12.1992**

(54) **Procédé et appareil de surveillance du déroulement de l'hémodialyse**

Verfahren und Vorrichtung zur Überwachung eines Hämodialysevorganges

Method and apparatus for monitoring the progress of hemodialysis

(84) Etats contractants désignés:
**AT BE CH DE DK ES FR GB IE IT LI NL SE**

(30) Priorité: **07.01.1992 FR 9200063**
**06.02.1992 FR 9201333**

(43) Date de publication de la demande:
**14.07.1993 Bulletin 1993/28**

(73) Titulaire: **LABORATOIRE EUGEDIA**
**F-60230 Chambly (FR)**

(72) Inventeurs:
• **Baudin, Serge**
**F-41190 Herbault (FR)**

• **Jussiaux, Philippe**
**F-60230 Chambly (FR)**

(74) Mandataire: **Michelet, Alain et al**
**Cabinet Harlé et Phelip**
**21 rue de la Rochefoucauld**
**F-75009 Paris (FR)**

(56) Documents cités:
**EP-A- 0 029 793**      **EP-A- 0 089 003**
**EP-A- 0 097 366**      **FR-A- 2 612 712**

• **PATENT ABSTRACTS OF JAPAN vol. 13, no. 124
(C-580)27 Mars 1989 & JP-A-63 294866 1
Décembre 1988**

## Description

L'invention concerne les techniques de contrôle de l'hémodialyse et plus particulièrement celles utilisant la mesure de l'hématocrite du sang du patient.

L'hématocrite est le rapport du volume occupé par les globules rouges au volume sanguin total.

Chez les malades victimes d'une insuffisance rénale, on fait appel au rein artificiel pour purifier leur sang et pour ajuster le volume hydrique de l'organisme à la valeur voulue. L'invention concerne plus particulièrement ce deuxième mécanisme où l'on utilise dans le dialyseur le phénomène d'ultra-filtration au travers d'une membrane semi-perméable. Au cours de cette opération, du liquide est extrait du plasma du malade et ce même plasma, en équilibre avec le tissu interstitiel et les cellules de l'organisme extrait à son tour du liquide de ceux-ci. Si l'opération est menée trop rapidement par rapport à ce que l'organisme peut supporter, apparaît une "hypovolémie plasmatique". Ce sont ces phénomènes que l'invention se propose de contrôler.

La diminution de la volémie pendant les séances de dialyse, résultante de phénomènes complexes d'échanges pluri-compartimentaux, est l'un des facteurs les plus importants de l'intolérance hémodynamique de cette méthode thérapeutique. Après une phase de latence plus ou moins longue, due à la mise en oeuvre de phénomènes biologiques de compensation, cette hypovolémie va souvent entraîner des systèmes cliniques de collapsus cardio-vasculaires, parfois bénins, parfois impressionnants, voire graves chez des patients fragiles. Il existe certes des moyens thérapeutiques ou techniques préventifs et/ou curatifs; le problème est de savoir quand et comment les utiliser à bon escient.

Il est bien établi, par l'expérience accumulée et par des travaux déjà anciens, que la surveillance discontinue, même très fréquente, de la pression artérielle et du rythme cardiaque ne permet pas dans de nombreux cas de dépister suffisamment tôt les incidents, du fait probablement du caractère soudain de l'effondrement des systèmes de compensation.

L'invention se donne pour tâche de fournir une méthode de suivi du déroulement de l'hémodialyse qui permette de prévoir assez tôt une insuffisance de la compensation par l'organisme de l'hypovolémie de manière à permettre au personnel de surveillance d'intervenir suffisamment tôt et d'éviter ainsi tout collapsus cardio-vasculaire.

La méthode doit être simple de mise en oeuvre, sûre et non traumatisante pour le patient.

Différentes méthodes de suivi de l'hématocrite pendant l'hémodialyse ont été proposées, plusieurs utilisaient la colorimétrie qui permet de suivre directement la concentration des hématies dans le sérum.

La mesure en continu de l'impédance du corps entier a été utilisée par plusieurs auteurs (TENDER, De VRIES, SCANFERLA) pour contrôler la dialyse, avec un certain succès. Mais l'appareillage utilisé est onéreux, gêne le patient et nécessite une relative immobilité. De plus, la méthode est peu fiable car les qualités bioélectriques des membranes cellulaires sont souvent très perturbées chez le dialysé.

Une méthode de mesure de la volémie, a été proposée dans le brevet européen EP-0.272.414 B. Dans ce document, on propose d'appliquer des formules compliquées dans lesquelles interviennent la conductivité du dialysat frais, le débit sanguin, les caractéristiques de puissance du dialyseur, pour déterminer d'abord la conductivité du plasma et celle du sang puis, à partir de celles-ci pour déterminer en continu l'hématocrite d'où l'on déduit la volémie ce qui permet de contrôler les paramètres de la dialyse. Cette méthode qui est très compliquée nécessite de nombreux étalonnages pour être fiable. Les formules standard utilisées dans les calculs ne sont pas en général adaptées à tous les patients. Enfin, les méthodes de ce type ne laissent que peu de place à l'intervention du praticien qui reste pourtant le seul responsable des opérations.

La méthode de l'invention, en comparaison, fournit des indications précises sur l'évolution de l'hématocrite. Elles permettent au praticien de prévoir, et dont de prévenir les réactions de l'organisme du patient.

Une méthode mixte utilisant à la fois une mesure de l'impédance corporelle et une mesure de l'impédance du sang en amont de l'appareil d'hémodialyse a été proposée dans le brevet européen EP-O.O29.793 B. La première de ces mesures s'effectue en implantant des aiguilles dans une main et un pied du patient. Celles-ci servent d'électrodes pour deux courants, l'un a une fréquence de 1 MHz et l'autre à 5 kHz. Grâce à ces courants, on détermine deux impédances. Une deuxième paire d'électrodes installée sur le circuit sanguin externe, en amont du dialyseur, permet également une mesure d'impédance - 5 kHz ici. La comparaison de ces trois valeurs d'impédance permet au praticien de suivre le déroulement de la dialyse. Il est également prévu de fixer une limite inférieure à l'impédance mesurée sur le circuit sanguin externe. Si cette limite est atteinte, une alarme est déclenchée. L'appareillage, objet du brevet EP-0.029.793.B permet au praticien de suivre le déroulement de l'hémodialyse. Cependant, si l'on désire l'exploiter à la seule fin de libérer le praticien d'une surveillance très étroite en utilisant le système d'alarme lié au seuil atteint par l'impédance du circuit externe, il aura fallu au préalable l'étalonner pour chaque patient. Il a par ailleurs été prouvé que ce seuil limite est atteint très tard, pratiquement au même moment que la diminution de la tension artérielle elle-même peut être constatée.

Par ailleurs, la méthode du brevet EP-0.029.793 B fait subir au patient, déjà traumatisé par l'hémodialyse, des piqûres supplémentaires qu'il serait préférable d'éviter.

Les deux techniques précédentes ont en commun l'inconvénient d'associer par le calcul les résultats de plusieurs mesures. Celles-ci, comme toutes les mesures biologiques ont leur domaine d'incertitude. En les introduisant dans des calculs compliqués, on risque de cumuler ces erreurs et d'aboutir à des conclusions aberrantes et qui risquent de compliquer le travail de surveillance du praticien.

Enfin, le document EP-A-0.089.003 décrit un appareil de purification de sang conçu de manière à surveiller le déroulement de l'hémodialyse. Cet appareil connu comprend une cellule de mesure destinée à être placée dans un circuit d'hémodialyse, un générateur de courant constant et un tube semi-rigide comportant deux électrodes reliées chacune à la source de courant. L'appareil connu comprend également un calculateur.

Compte-tenu de la définition de l'hématocrite donnée plus haut, on peut accéder à sa mesure soit par la mesure du taux de globules rouges soit par la mesure du volume plasmatique. Les systèmes électroniques associés aux capteurs effectuent les conversions nécessaires. C'est la raison pour laquelle, dans ce qui suit, le terme hématocrite désigne soit l'hématocrite proprement dite, soit le taux de globules rouges (hérythrocyte), soit le volume plasmatique.

L'invention concerne un procédé de surveillance du déroulement de l'hémodialyse par mesure extra corporelle de l'hématocrite defini dans la revendication 1.

Lorsque la variation sur la courte durée est supérieure à la variation sur la moyenne durée, l'indicateur augmente la valeur qu'il indique et il la décroît lorsque la variation sur la courte durée est inférieure à celle sur la moyenne durée. Avantageusement, la moyenne durée est de l'ordre du triple de la courte durée et de préférence d'environ 15 minutes et la combinaison du premier et du second indicateurs du déroulement de l'hémodialyse constitue le niveau d'alarme. De préférence, la mesure de l'hématocrite résulte d'une mesure de l'impédance sanguine, et l'impédance mesurée est le résultat de mesures instantanées moyennes effectuées de préférence à une fréquence de 5 kHz.

L'invention concerne également l'appareil qui permet la mise en oeuvre du procédé et qui comporte une cellule de mesure destinée à être placée dans un circuit d'hémodialyse, un générateur de courant constant, un impédancemètre, ladite cellule de mesure comportant un tube semi-rigide muni de deux ouvertures fermées par des capsules et reliées chacune à la source de courant d'une part, et à l'impédancemètre d'autre part, et qui comporte un calculateur recevant le signal fourni par l'impédancemètre et commandant un indicateur du déroulement de l'hémodialyse.

L'intérêt essentiel de la méthode et du dispositif de l'invention est de permettre la détection précoce de l'hypovolémie, bien avant toute manifestation traumatisante et de permettre ainsi de protéger le patient de tout risque de collapsus.

La simplicité de la méthode permet la réalisation d'appareils peu compliqués et donc relativement bon marché, ce qui facilitera leur large diffusion.

L'exploitation informatique des résultats permet leur utilisation aisée dans le cadre des recherches médicales.

La description et les figures qui suivent permettront de comprendre le fonctionnement de l'invention et d'apprécier ses avantages.

La Figure 1 montre l'évolution de la variation relative du volume plasmatique dans le temps.

La Figure 2 représente la cellule de mesure d'impédance implantée sur le circuit sanguin externe.

La Figure 3 schématise la mesure d'impédance; quant à la Figure 4, elle représente le schéma fonctionnel de la série d'opérations réalisées par le dispositif de l'invention.

La Figure 5 montre l'évolution comparée de la volémie d'un patient et de sa tension artérielle.

La Figure 6 montre un exemple de réalisation de l'appareil de l'invention.

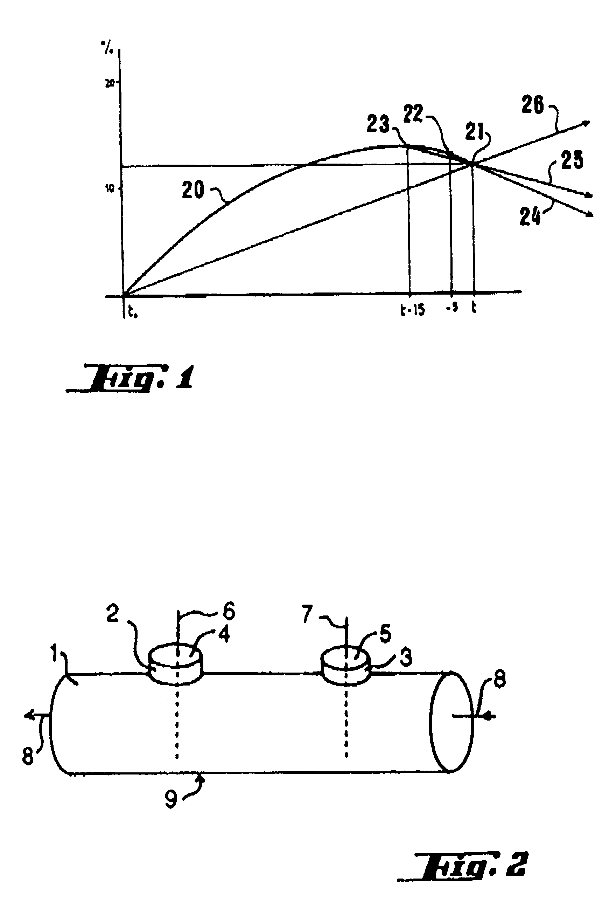

Sur la Figure 1, on a représenté l'évolution de la variation relative de l'hématocrite telle qu'elle peut être déduite d'une mesure faite en temps réel sur le sang d'un patient au cours d'une séance d'hémodialyse.

L'évaluation de l'hématocrite peut se faire par exemple par mesure colorimétrique ou, selon le mode de réalisation préféré de l'invention, par impédancemétrie. On peut aussi associer une mesure colorimétrique à une mesure par impédancemétrie.

La fonction de l'hématocrite considérée selon l'invention peut être de différents types. On considère maintenant la variation relative du volume plasmatique:

$$\frac{VPo - VP}{VPo} = -\frac{\Delta VP}{VPo} \ (t)$$

Sur la Figure 1, on voit en abscisses le temps en minutes et en ordonnées, la valeur

$$\frac{-\Delta V \ P}{VPo} x \ 100$$

qui est, en pourcentage, la variation relative du volume plasmatique depuis l'instant $t_o$ jusqu'à l'instant t. La courbe 20 peut être obtenue à partir des mesures effectuées par l'appareil de l'invention.

A un instant donné t, on considère trois valeurs de

$$\frac{-\Delta VP}{VPo}$$

la valeur à l'instant t (point 21 de la courbe), celle à l'instant qui le précède de 5 minutes (point 22) et celle mesurée à l'instant qui le précède de 15 minutes (point 23). Le principe de la mesure de l'invention consiste à comparer l'évolution de la pente de la courbe 20 sur la courte durée (ici 5 minutes) matérialisée par la droite 24 par rapport à celle de la pente sur la moyenne durée (ici 15 minutes) matérialisée par la droite 25 et sur la longue durée (depuis le début de la dialyse) droite 26.

Les méthodes colorimétriques qui permettent de suivre le taux de globules rouges du sang sont bien connues. La méthode préférée ici est celle où source lumineuse et détecteur se trouvent d'un même côté de la cellule où circule le sang, elle permet une mesure de la lumière rétrodiffusée et est ainsi moins sensible aux artéfacts. Mais la méthode électrique d'évaluation indirecte de l'hématocrite à partir d'une mesure de l'impédance sanguine est la plus pratiquée et la plus facile à mettre en oeuvre. C'est elle qui sera décrite en détail ci-dessous.

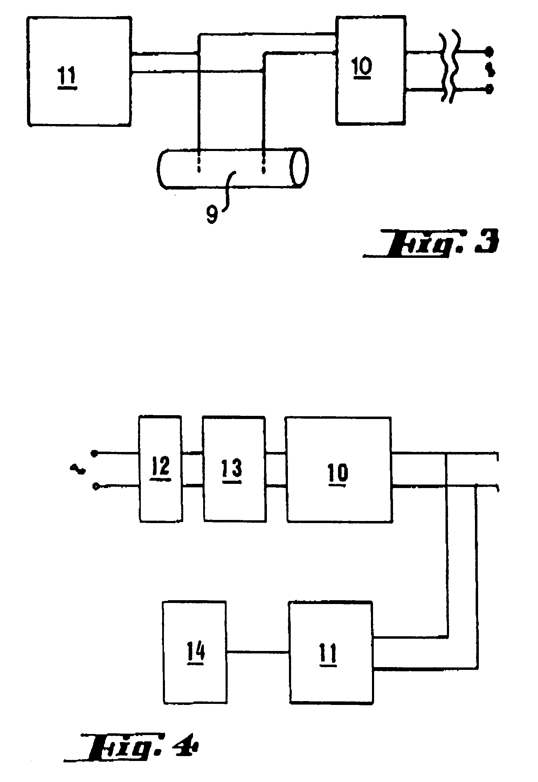

Sur la Figure 2, on voit un tube semi-rigide I en matière plastique avec une section de surface bien définie, par exemple 20 mm$^2$. A deux emplacements distants d'un espace de - typiquement - 70 mm sont deux capsules 2, 3 fermées par un matériau élastomère en 4, 5 dans lequel sont plantées les électrodes 6, 7. Ces électrodes sont par exemple deux aiguilles stériles introduites au travers des membranes 4, 5 qui se referment sur elles de manière parfaitement étanche. Ces membranes constituent les parois latérales du tuyau I dans lequel le sang 8 du malade circule. L'ensemble est stérile et de préférence jetable. Cette cellule de mesure 9 est placée de préférence à l'entrée du système d'hémodialyse. Ainsi la température de la cellule reste sensiblement constante, elle est à peine inférieure à la température du patient.

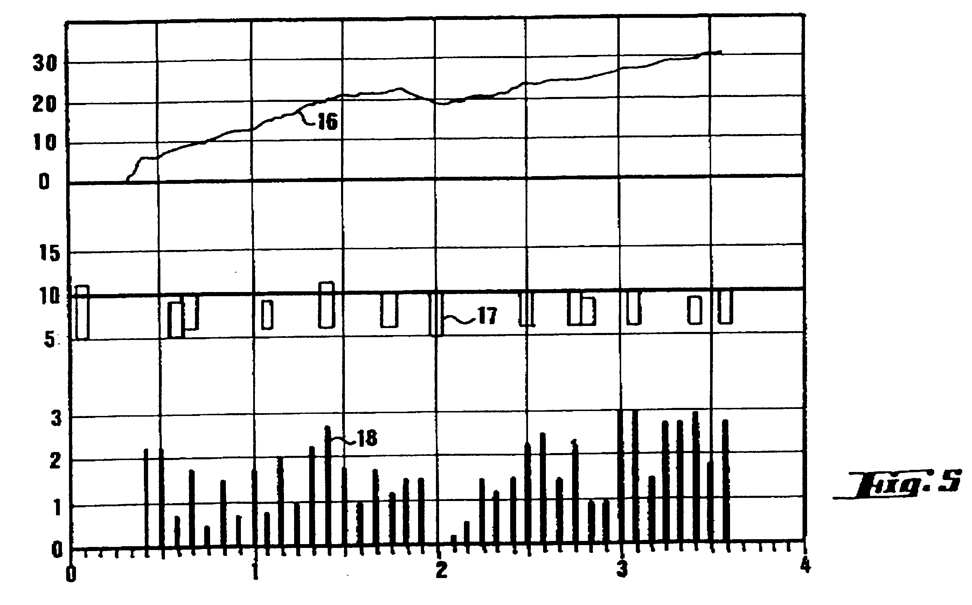

Sur la Figure 3 est représentée la même cellule 9 alimentée par un générateur de courant constant 10. Ce courant est sinusoïdal d'une fréquence de 5 kHz. Grâce à l'impédancemètre II, la mesure de la différence de potentiel entre les électrodes 6, 7 à un instant donné fournit une mesure de l'impédance Z du sang à cet instant.

Sur la Figure 3, on a représenté le schéma fonctionnel complet de la chaîne de mesures. On voit en 12 un circuit de protection destiné à éviter tout risque de liaison directe entre l'alimentation en courant alternatif du secteur et l'appareil - spécialement ses électrodes en contact avec le sang du patient. En 13, figure l'alimentation basse tension à une fréquence de 5 kHz et en 10 le générateur de courant constant qui alimente les électrodes tandis que l'impédancemètre II calcule l'impédance entre les électrodes 6, 7 à partir de la chute de la tension alternative mesurée entre elles, à sa sortie. On dispose d'un signal proportionnel à l'impédance instantanée Z. Le calculateur 14 effectue les relevés de ces mesures à une cadence de n par secondes, par exemple 5. Le calculateur 14 qui fait partie d'un microprocesseur effectue tout d'abord un lissage des mesures instantanées, il calcule la moyenne de Z sur p secondes, par exemple sur 5 secondes. Dans l'exemple, la mesure est donc moyennée sur 25 mesures élémentaires. On obtient ainsi une valeur Zm. C'est cette mesure qui va servir à suivre l'évolution de l'impédance dans le temps.

L'impédance Z du sang mesurée à 5 kHz (qui est l'équivalent de la résistance électrique mesurée en courant continu) est proportionnelle au taux de globules rouges dans le sang et donc à l'hématocrite (rapport du volume occupé par les globules rouges au volume sanguin total).

L'appareil de l'invention est mis en marche cinq minutes environ après le début de la séance d'hémodialyse. Il détermine une première valeur de $Z_o$. Celle-ci, grâce à un étalonnage préalable, permet un calcul de l'hématocrite correspondante. Une estimation du volume sanguin total du patient effectuée par le praticien permet d'en déduire le volume plasmatique de départ $VP_o$ (le volume plasmatique + le volume des globules rouges = le volume sanguin total ).

A partir de l'instant $t_o$ du début de la mesure, l'impédance Z est mesurée en permanence et mise en mémoire. Les valeurs utiles au suivi de l'hémodialyse seront prises dans la mémoire selon les besoins.

Deux grandeurs sont utiles à ce suivi, d'une part la variation relative de l'hématocrite (qui est au signe près, sensiblement égale à la variation relative de volume plasmatique) et d'autre part, le niveau de cette hématocrite (ou de ce volume plasmatique) qui est proportionnelle à l'impédance. Chacune de ces deux grandeurs sera analysée et permet d'évaluer indépendamment un indicateur du déroulement de l'hémodialyse. La combinaison de ces deux indicateurs permet, elle, de déterminer un niveau d'alerte.

La méthode de l'invention est mise en oeuvre à intervalles réguliers au cours des séances d'hémodialyse, par exemple toutes les cinq minutes. La figure I représente les grandeurs qui sont calculées à chaque mise en oeuvre: ce sont d'une part, la pente sur la courte durée PCD représentée par la droite 24 et la pente sur la moyenne durée PMD (droite 25) et la pente sur la longue durée PLD, pour le premier indicateur du déroulement (l'indicateur de pente; IP). D'un autre côté, pour calculer l'indicateur de seuil IS, on utilise la valeur de l'ordonnée du point 21 lui-même.

L'indicateur de pente IP est la somme de trois grandeurs, l'une, $A_I$ qui dépend de la pente sur la courte durée PCD, l'autre, $A_2$, de la pente sur la moyenne durée PMD et la troisième, $A_3$, qui est liée à la très longue durée, PLD (droite 26 de la Figure I).

Ces trois pentes s'expriment en pourcentages de variation de volume plasmatique ramenés à une minute et affectés du signe moins.

Le terme $A_3$ est toujours la même fonction de la pente sur la longue durée:

$$A_3 = \left| \frac{PLD}{10} \right|$$

(valeur absolue arrondie à l'entier voisin)

Les deux autres termes $A_1$ et $A_2$ sont composés de deux parties, l'une qui ne dépend que de la pente et l'autre qui résulte de la comparaison de celle-ci avec la pente sur la durée immédiatement supérieure.

L'expression de $A_2$ est ainsi:

$$A_2 = \left| \frac{PMD}{15} \right| + \text{constante}$$

$$\left| \frac{PMD}{15} \right|$$

étant la valeur absolue arrondie au nombre entier voisin, cette constante est de +1 si

$$PMD < PLD - 4$$

et de - 1 si

$$PMD > PLD + 4$$

De même, l'expression de $A_1$ est:

$$A_1 = \left| \frac{PCD}{20} \right| + \text{constante}$$

$$\left| \frac{PCD}{20} \right|$$

étant la valeur absolue arrondie à l'entier voisin;
la constante est égale à +2 si:

$$PCD < PMD - 4$$

et égale à -2 si:

$$PCD > PMD + 4$$

On voit ainsi que l'indicateur de pente IP s'exprime toujours par un nombre entier, positif ou négatif. Le microprocesseur de l'appareil a pour mission de garder IP à l'intérieur de la fourchette -9, +9 (les valeurs extérieures à ce domaine sont en effet toujours dues à des artéfacts, l'expérience l'a prouvé).

L'indicateur de seuil IS quant à lui s'exprime par:

$$\left( \frac{\frac{-\Delta VP}{VP_o} \times 100}{100} \right)^2$$

Cette valeur est, elle aussi arrondie à l'entier voisin. Pour les mêmes raisons que précédemment, elle est limitée à la fourchette, 0 + 9.

Le niveau d'alarme (NA) est égal à l'addition des deux indicateurs précédents:

$$NA = IP + IS$$

$$-9 < NA < + 18$$

Les valeurs positives sont les seules qui soient alarmantes.

Selon les caractéristiques des malades (corpulence, état général , etc) on fixe une limite à NA qui est soit 4, soit 8, soit 12. L'appareil est construit de telle manière que si la limite fixée est dépassée, une alarme sonore se déclenche.

Comme on l'a vu, IS à lui seul ne permet pas de prévenir les incidents dans le déroulement de la dialyse. En revanche, IP fournirait à lui seul des indications exploitables directement. Sa combinaison avec IS présente l'intérêt de permettre une analyse encore plus fine et donc, une prévision encore meilleure.

Dans le cas où l'hématocrite est déterminée par mesure de l'impédance, l'inconvénient de la méthode pourrait être que la variation d'impédance peut résulter soit de la variation de volémie, soit de celle de la concentration des électrolytes dans le plasma, en particulier du sodium, mais ces dernières variations sont lentes et interviennent peu dans la comparaison des pentes sur des durées brèves.

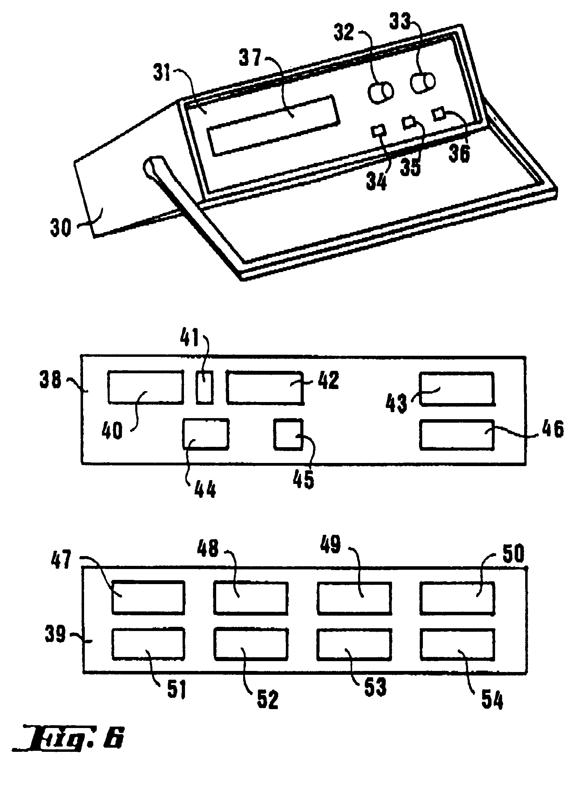

L'appareil de l'invention est représenté Figure 6. Il s'agit d'un modèle avec indication des volumes plasmatiques VP. On voit en 30 le boîtier sur la face avant 31 duquel sont disposés les différents organes utiles à la mesure. Il s'agit du sélecteur de mode 32 à quatre positions, du sélecteur d'alarme 33 à trois positions, du poussoir 34 pour la sélection des pages d'écran, du poussoir 35 de départ de cycle de mesure ainsi que de l'interrupteur d'alarme 36.

En 37, on a représenté l'écran LCD. Cet écran permet l'affichage au choix (poussoir 34) de l'une des deux pages, la page 1 (38) ou la page 2 (39). Sur la page 1, on voit s'afficher:

en 40, l'impédance initiale en Kiloohms (temps $t_o$)

en 41, l'indicateur d'incident dans le fonctionnement,

en 42, l'impédance au temps t,

en 43, PCD au temps t,

en 44 valeur de IP, l'indicateur de pente,

en 46, valeur de IS, l'indicateur de seuil,

en 46, valeur du volume plasmatique VP au temps t.

Si c'est la page 2 qui est sélectionnée, on y voit apparaître:

en 47, la pente sur la longue durée (PLD) depuis l'origine $t_o$,

en 48, PCD à t-10 minutes,

en 49, PCD à t-5 minutes,

en 50, PCD au temps t (comme en 43, page 1),

en 51, volume plasmatique initial $VP_o$ (en ml)

en 52, variation absolue de VP à t-10 mn,

en 53, variation absolue de VP à t-5 mn,

en 54, variation absolue de VP à t (comme en 46).

Toutes les indications de l'appareil de la figure 6 sont en volumes plasmatiques. Il s'agit d'une grandeur facile à exploiter par le praticien. La valeur du volume plasmatique se déduit comme on l'a vu de la valeur mesurée de l'hématocrite, elle-même déterminée à partir d'une mesure d'impédance ou d'une mesure colorimétrique ou de toute autre méthode qui permet d'accéder au taux de globules rouges dans le sang du patient.

Une mesure s'effectue de la manière suivante:

. L'appareil est mis sous tension.

. Les électrodes sont connectées - immédiatement la mesure de l'impédance effectuée toutes les cinq secondes s'affiche en 42.

. Avec le sélecteur de mode 32, on choisit soit le mode manuel (mesure d'impédance seule) soit l'une des trois positions du mode automatique $A_I$, $A_2$ ou $A_3$.

$A_1$ correspond à un patient de faible corpulence (volume sanguin supposé 3500 ml)

A2 corpulence moyenne (4000 ml)

$A_3$ corpulence forte (4500 ml)

. Avec le sélecteur d'alarme 33, on sélectionne le niveau pour la somme (IS + IP) pour lequel l'alarme doit se déclencher. Dans le cas décrit précédemment trois niveaux étaient prévus: 4, 8 et 12 (sensibilité forte, moyenne et faible respectivement).

. Dès que les paramètres de la dialyse sont stabilisés, en général cinq minutes après le début, on appuie sur le poussoir 35.

Si l'appareil est en mode automatique (position $A_1$, $A_2$ ou $A_3$ du bouton 32) le déroulement des mesures est le suivant:

- de 0 a 10 mn:    observation de la valeur de départ pondérée de l'impédance

- à la $10^e$ mn:    calcul et affichage de $Z_o$ (40) et du volume plasmatique de départ (51)

- a la 15$_e$ mn: calcul et affichage de la première variation du volume plasmatique (46 et 54) et pente sur la courte durée PCD (43 et 50) qui est exceptionnellement la même que PLD (47).

- ensuite, toutes les 5 mn - calcul et affichage de toutes les valeurs, en particulier IP et IS et déclenchement de l'alarme si

IP + IS $\geq$ seuil d'alarme
(tel que sélectionné en 33)

L'exemple suivant compare l'évolution de la mesure de l'hématocrite extra-corporelle ou celle de son corollaire inverse, la volémie, à l'évolution de la tension artérielle du patient, il montre que la méthode de l'invention permet de prévoir les troubles chez le patient au moins un quart d'heure avant qu'ils ne se produisent et donne ainsi au praticien la possibilité de les éviter.

Sur la Figure 5, on a illustré l'exemple. On voit sur l'axe des abscisses, le temps exprimé en heures. L'axe des ordonnées montre dans sa partie médiane 17, les valeurs des tensions artérielles exprimées en centimètres de mercure et, dans la partie supérieure 16, la variation relative du volume plasmatique

$$- \frac{\Delta \, \text{VP}}{\text{VP}}$$

exprimé en %.

Il s'agit d'une grandeur proportionnelle à l'impédance Zm au même instant, celle qui a permis d'établir les niveaux d'alerte inscrits en bas du diagramme, en 18. Ces niveaux d'alerte, égaux à IP+IS étaient compris entre 0 et 12. Les seuils sont, comme on l'a vu, fixés respectivement à 4, 8 et 12.

Sur la figure, on peut suivre la correspondance entre l'hématocrite (en l'occurrence, le volume plasmatique), le niveau d'alarme et la tension artérielle. On voit en particulier que, lorsque la pente de la courbe de l'hématocrite augmente, le niveau d'alarme s'accroît et que, peu après, la tension du malade diminue. C'est ainsi qu'à 25 minutes, la pente s'accroît fortement, que le niveau d'alarme dépasse le seuil n° 2 (valeur de 9). C'est un quart d'heure plus tard que la tension est tombée. De même à 2h 35, le niveau 10 a été atteint et il a été suivi d'une amélioration puis de nouveau, d'un niveau inquiétant à 2h 45, suivi lui-même 5 minutes plus tard d'une chute de tension.

Il est à remarquer que l'expérience a été faite sans l'intervention du praticien alors que, normalement, dès la première alarme, il aurait agi, soit en ralentissant l'ultrafiltration, soit par l'injection de solutés qui provoquent un effet d'expansion plasmatique (effet osmotique).

Sur la Figure 5, aucune mesure n'a été prise malgré les premières alarmes dues à la pente seule. C'est pourquoi, dans la suite, l'effet du niveau de l'hématocrite a été déterminant sur les alarmes ultérieures.

La méthode de suivi de l'hématocrite du sang du patient pendant l'hémodialyse qui vient d'être décrite utilisait essentiellement la mesure de l'impédance. En effet, les mesures électriques sont faciles à mettre en oeuvre et à informatiser. Cependant, toute méthode qui permet de suivre l'évolution d'une grandeur proportionnelle à l'hématocrite ou à la volémie convient pour mettre en l'oeuvre l'invention. Il en est ainsi des méthodes optiques comme la colorimétrie par exemple qui permet une évolution très précise de la concentration du sang en hématies et donc de l'hématocrite.

Par rapport aux techniques antérieures, l'invention offre l'avantage de permettre une détection de l'hypovolémie très tôt, bien avant que la manifestation physique, jusqu'à présent la plus facile à détecter, l'hypotension artérielle, se manifeste. Dans le cas des patients au comportement atypique, elle permet dès la première dialyse consécutive à une dialyse où un incident s'est présenté, de prévenir avec une grande sécurité tout nouvel incident. La mise en oeuvre de la technique de l'invention est simple, pas traumatisante pour le patient puisque son dispositif s'intègre facilement au rein artificiel lui-même. Les résultats de la méthode de l'invention se sont avérés par ailleurs indépendants des variations de la natrémie (taux de sodium dans le sang). Le maniement de l'appareil est particulièrement simple et ne nécessite qu'un apprentissage réduit pour le praticien souhaitant le mettre en oeuvre. De plus, dans le cas des patients à risque ou pour procéder à des recherches, il est toujours possible de suivre en temps réel le déroulement de la dialyse. De même, il est possible d'enregistrer et de stocker les données par des moyens informatiques et de permettre ainsi aussi bien le suivi de l'évolution d'un malade, que des études à caractère scientifique - éventuellement statistiques - d'une plus grande ampleur. La méthode est simple sur le plan électrique - électronique, le prix de revient de chaque dispositif est donc limité et il est possible d'équiper chaque centre d'hémodialyse du nombre d'appareils nécessaires.

**Revendications**

1. Procédé de surveillance du déroulement de l'hémodialyse par mesure extra-corporelle de l'hématocrite utilisant un indicateur d'alarme (NA) évalué selon les étapes suivantes:

- on mesure à chaque instant une grandeur proportionnelle à l'hématocrite,
- on détermine à chaque instant les variations de cette grandeur sur une courte durée (PCD), sur une moyenne durée (PMD) et sur une longue durée (PLD) précédant cet instant et on ramène ces variations à la même unité de temps obtenant ainsi des valeurs ($A_1$, $A_2$, $A_3$) proportionnelles aux variations desdites grandeurs (PCD, PMD, PLD), et
- on calcule un indicateur de pente (IP) en combinant les variations desdites grandeurs (PCD, PMD, PLD), l'indicateur de pente (IP) étant d'autant plus grand que l'hématocrite baisse plus rapidement,
- c'est l'indicateur de pente (IP), combiné éventuellement à un indicateur de seuil (IS) fonction de l'hématocrite, qui constitue l'indicateur d'alarme (NA).

2. Procédé selon la revendication 1, caractérisé en ce que, lorsque la grandeur proportionnelle à l'hématocrite subit une variation sur la courte durée (PCD) sensiblement inférieure à sa variation sur la moyenne durée (PMD), l'indicateur de pente s'accroît et en ce qu'il décroît dans le cas contraire.

3. Procédé selon la revendication 1 ou la revendication 2, caractérisé en ce que, lorsque la grandeur proportionnelle à l'hématocrite subit une variation sur la moyenne durée (PMD) sensiblement inférieure à sa variation sur la longue durée (PLD), l'indicateur de pente s'accroît et en ce qu'il décroît dans le cas contraire.

4. Procédé selon l'une des revendications précédentes, caractérisé en ce que la longue durée est le temps écoulé depuis le début de l'hémodialyse et en ce que la moyenne durée est de l'ordre du triple de la courte durée.

5. Procédé selon la revendication 4, caractérisé en ce que dans l'indicateur de pente (IP), la grandeur (PLD) intervient de l'ordre de deux fois plus que la grandeur (PCD et de l'ordre de une fois et demie plus que la grandeur (PMD).

6. Procédé selon la revendication 5, caractérisé en ce que dans l'indicateur de pente (IP), une variation relative donnée de la grandeur de (PCD) par rapport à la grandeur (PMD) intervient de l'ordre de deus fois plus que la même variation relative de la grandeur de (PMD) par rapport à la grandeur (PLD).

7. Procédé selon l'une des revendications précédentes, caractérisé en ce que l'indicateur de seuil (IS) est proportionnel au carré de la variation relative de l'hématocrite depuis le début de l'hémodialyse.

8. Procédé selon la revendication 7, caractérisé en ce que l'indicateur d'alarme (NA) est la somme de l'indicateur de pente (IP) et de l'indicateur de seuil (IS).

9. Procédé selon l'une des revendications précédentes, caractérisé en ce que la mesure de l'hématocrite résulte d'une mesure de l'impédance sanguine.

10. Procédé selon la revendication 9, caractérisé en ce que l'impédance mesurée est le résultat de mesures instantanées moyennées effectuées de préférence à une fréquence de 5 kHz.

11. Appareil de surveillance du déroulement de l'hémodialyse par mesure extra-corporelle de l'hématocrite conformément au procédé de l'une quelconque des revendications 1 à 10, l'appareil comportant une cellule de mesure (9) destinée à être placée dans un circuit d'hémodialyse, un générateur de courant constant (10), une impédancemètre (11), ladite cellule de mesure (9) comportant un tube semi-rigide (1) muni de deux ouvertures fermées par des capsules (2, 3), deux électrodes (6, 7) traversant lesdites capsules et reliées chacune à la source de courant (10), d'une part, et à l'impédancemètre d'autre part, caractérisé en ce qu'il comporte un calculateur (14) recevant le signal fourni par l'impédancemètre (11) et commandant un indicateur du déroulement de l'hémodialyse.

12. Appareil selon la revendication 11, caractérisé en ce que la source de courant (10) est alimentée à partir du secteur par un circuit de protection (12).

13. Appareil selon l'une quelconque des revendications 11 et 12, caractérisé en ce que la cellule de mesure (9) est placée à l'entrée du dispositif d'hémodialyse.

14. Appareil selon l'une quelconque des revendications 11 à 13, caractérisé en ce que le générateur de courant fournit un courant ayant une fréquence égale à 5 kHz.

**Claims**

1.  Method of monitoring the progress of hemodialysis by extra-corporeal measurement of the hematocrit, using an alarm level (NA) which is determined by the following steps:

    -   measuring at a given time a quantity which is proportional to the hematocrit,
    -   determining at a given time the variations of that quantity over a short period (PCD), a medium period (PMD), and a long period (PLD) preceding that time, normalizing these variations to the same unit of time and thereby obtaining values ($A_1$, $A_2$, $A_3$) which are proportional to the variations of the quantities (PCD, PMD, PLD), and
    -   calculating a slope indicator (IP) by combining the variations of the three quantities (PCD, PMD, PLD), where the slope indicator (IP) is all the greater the more quickly the hematocrit decreases;
    -   the slope indicator (IP), possibly combined with a threshold indicator (IS) which is a function of the hematocrit, constitutes the alarm level (NA).

2.  Method as claimed in Claim 1, characterized in that the slope indicator increases when the quantity which is proportional to the hematocrit varies less over the short period (PCD) than over the medium period (PMD), and in that it decreases in the opposite case.

3.  Method as claimed in Claim 1 or 2, characterized in that the slope indicator increases when the quantity which is proportional to the hematocrit varies less over the medium period (PMD) than over the long period (PLD), and in that it decreases in the opposite case.

4.  Method as claimed in one of the preceding Claims, characterized in that the long period is the time elapsed since the beginning of the hemodialysis and in that the medium period is about three times as long as the short period.

5.  Method as claimed in Claim 4, characterized in that in the calculation of the slope indicator (IP) the quantity (PLD) is weighted twice as much as the quantity (PCD) and one and a half times as much as the quantity (PMD).

6.  Method as claimed in Claim 5, characterized in that in the calculation of the slope indicator (IP) a given relative variation of the quantity (PCD) relative to the quantity (PMD) is weighted twice as much as the same relative variation of the quantity (PMD) relative to the quantity (PLD).

7.  Method as claimed in one of the preceding Claims, characterized in that the threshold indicator (IS) is proportional to the square of the relative variation of the hematocrit since the beginning of the hemodialysis.

8.  Method as claimed in Claim 7, characterized in that the alarm level (NA) is the sum of the slope indicator (IP) and the threshold indicator (IS).

9.  Method as claimed in one of the preceding Claims, characterized in that the measurement of the hematocrit results from an impedance measurement of the blood.

10. Method as claimed in Claim 9, characterized in that the measured impedance results from averaged measurements at given times preferably implemented at a frequency of 5 kHz.

11. Apparatus for monitoring the progress of hemodialysis by extra-corporeal measurement of the hematocrit according to the method as claimed in any one of Claims 1 to 10, where the apparatus comprises a measurement cell (9) designed to be placed in a hemodialysis circuit, a constant-current generator (10), an impedance meter (11), said measurement cell comprising a semi-rigid tube (1) provided with two openings closed by caps (2, 3), two electrodes passing through said caps and each one being connected to the current source (10) on the one hand and to the impedance meter on the other hand, characterized in that it comprises a calculator (14) receiving the signal generated by the impedance meter and actuating an indicator of the progress of hemodialysis.

12. Apparatus as claimed in Claim 11, characterized in that the current source (10) is supplied from the mains via a protection circuit (12).

13. Apparatus as claimed in one of Claims 11 and 12, characterized in that the measurement cell (9) is placed at the inlet of the hemodialysis device.

**14.** Apparatus as claimed in one of Claims 11 to 13, characterized in that the current generator supplies a current with a frequency of approximately 5 kHz.


**Patentansprüche**

**1.** Verfahren zur Überwachung des Ablaufs einer Dialyse durch außerhalb des Körpers vorgenommene Messung des Hämatokrits unter Verwendung eines Alarmindikators (NA), der in folgenden Schritten ermittelt wird:

- man mißt laufend eine zum Hämatokrit proportionale Größe,
- man bestimmt laufend die Änderungen dieser Größe über eine einem jeweiligen Meßzeitpunkt vorangehende kurze Dauer (PCD), mittlere Dauer (PMD) und lange Dauer (PLD) und bezieht diese Änderungen auf eine einheitliche Zeiteinheit, wobei man Werte ($A_1$, $A_2$, $A_3$) erhält, die zu den Änderungen der Größen (PCD, PMD, PLD) proportional sind, und
- man errechnet einen Steigungsindikator (IP) durch Kombinieren der Änderungen der Größen (PCD, PMD, PLD), wobei der Steigungsindikator (IP) umso größer ist, je schneller der Hämatokrit sinkt;
- der Steigungsindikator (IP), eventuell mit einem vom Hämatokrit abhängigen Schwellwertindikator (IS) kombiniert, bildet dann den Alarmindikator (NA).

**2.** Verfahren nach Anspruch 1, dadurch gekennzeichnet, daß der Steigungsindikator größer wird, wenn die zum Hämatokrit proportionale Größe über die kurze Dauer (PCD) eine im wesentlichen kleinere Änderung erfährt als über die mittlere Dauer (PMD), und daß er im entgegengesetzten Fall kleiner wird.

**3.** Verfahren nach Anspruch 1 oder 2, dadurch gekennzeichnet, daß der Steigungsindikator größer wird, wenn die zum Hämatokrit proportionale Größe über die mittlere Dauer (PMD) eine im wesentlichen kleinere Änderung erfährt als über die lange Dauer (PLD), und daß er im entgegengesetzten Fall kleiner wird.

**4.** Verfahren nach einem der vorangehenden Ansprüche, dadurch gekennzeichnet, daß die lange Dauer die seit Beginn der Dialayse vergangene Zeit ist und daß die mittlere Dauer etwa das Dreifache der kurzen Dauer beträgt.

**5.** Verfahren nach Anspruch 4, dadurch gekennzeichnet, daß die Größe (PLD) etwa doppelt so stark wie die Größe (PCD) und etwa anderthalb mal so stark wie die Größe (PMD) in den Steigungsindikator (IP) eingeht.

**6.** Verfahren nach Anspruch 5, dadurch gekennzeichnet, daß eine gegebene relative Änderung der Größe (PCD) gegenüber der Größe (PMD) etwa doppelt so stark in den Steigungsindikator (IP) eingeht wie die gleiche relative Änderung der Größe (PMD) gegenüber der Größe (PLD).

**7.** Verfahren nach einem der vorstehenden Ansprüche, dadurch gekennzeichnet, daß der Schwellwertindikator (IS) proportional zum Quadrat der relativen Änderung des Hämatokrits seit Beginn der Dialyse ist.

**8.** Verfahren nach Anspruch 7, dadurch gekennzeichnet, daß der Alarmindikator (NA) die Summe aus dem Steigungsindikator (IP) und dem Schwellwertindikator (IS) ist.

**9.** Verfahren nach einem der vorstehenden Ansprüche, dadurch gekennzeichnet, daß die Messung des Hämatokrits aus einer Messung der Impedanz des Bluts resultiert.

**10.** Verfahren nach Anspruch 9, dadurch gekennzeichnet, daß die gemessene Impedanz aus vorzugsweise mit einer Frequenz von 5 kHz vorgenommenen gemittelten Einzelmessungen resultiert.

**11.** Gerät zur Überwachung des Ablaufs einer Dialyse durch außerhalb des Körpers vorgenommene Messung des Hämatokrits nach dem Verfahren gemäß einem der Ansprüche 1 bis 10, wobei das Gerät eine zum Eingefügtwerden in einen Dialysekreislauf bestimmte Meßzelle (9), einen Konstantstromgenerator (10), einen Impedanzmesser (11) aufweist, wobei die Meßzelle (9) eine halbfeste Röhre (1) mit zwei durch Kapseln (2, 3) verschlossenen Öffnungen, zwei die Kapseln durchdringende Elektroden (6, 7), von denen jede einerseits mit der Stromquelle und andererseits mit dem Impedanzmesser verbunden ist, dadurch gekennzeichnet, daß es einen Rechner (14) aufweist, der das vom Impedanzmesser (11) gelieferte Signal empfängt und einen Indikator für den Ablauf der Dialyse steuert.

**12.** Gerät nach Anspruch 11, dadurch gekennzeichnet, daß die Stromquelle (10) vom Stromnetz über einen Schutzkreis (12) versorgt wird.

**13.** Gerät nach einem der Ansprüche 11 und 12, dadurch gekennzeichnet, daß die Meßzelle (9) an den Eingang der Dialysevorrichtung gelegt ist.

**14.** Gerät nach einem der Ansprüche 11 bis 13, dadurch gekennzeichnet, daß der Stromgenerator einen Strom mit einer Frequenz von 5 kHz liefert.

Fig. 1

Fig. 2

_Fig. 3_

_Fig. 4_

Fig. 5

EP 0 551 043 B1

**Fig. 6**